# EUROPEAN PATENT APPLICATION

(11) **EP 1 524 619 A2**
(43) Date of publication of application: **20.04.2005**
(21) Application number: 04256310.6
(22) Date of filing: 13.10.2004
(51) Int. Cl.: G06F 19/00

(54) **System and method for wireless data communication**

(30) Priority: 14.10.2003 US 511060 P
(71) Applicant: RF MONOLITHICS, INC., Dallas, Texas 75244 (US)
(72) Inventor: Kirk, David, McKinney Texas 75070 (US); Perkins, Frank H., Arlington Texas 76012 (US); Hinkle, Terry L., Plano Texas 75093 (US)
(74) Representative: Style, Kelda Camilla Karen

(57) **Abstract**

A system includes a database, a controller, and multiple nodes. The database is capable of storing information. The controller is capable of facilitating access to the database. The controller is also capable of detecting a new node in the network and, if necessary, communicating a network protocol to the new node to allow the new node to participate in the network.
The information in the database could represent medical-related information. The nodes could include fixed nodes and portable nodes. The network could represent a mesh network.

## Description

### CROSS REFERENCE TO RELATED APPLICATIONS

This application claims priority under 35 U.S.C. 119(e) to U.S. Provisional Patent Application No. 60/511,060 filed on October 14, 2003, which is hereby incorporated by reference.

### TECHNICAL FIELD

This disclosure is generally directed to data communication systems. More specifically, this disclosure is directed to a system and method for wireless data communications.

### BACKGROUND

Data communication systems have been used for many years. Conventional data communication systems typically include wired communication circuits that attach remote hardware to a central processing facility. At best, conventional data communication systems often have limited wireless communication capabilities with the remote hardware. Moreover, it is often costly and difficult to retrofit existing facilities having wired communication systems with wireless communication equipment.

### SUMMARY

This disclosure provides a system and method for wireless data communications.

In one aspect, a system includes a database capable of storing medical-related information. The system also includes a plurality of wireless nodes forming a mesh network. At least one of the plurality of wireless nodes is capable of communicating at least a portion of the medical-related information for storage in the database. At least one of the plurality of wireless nodes is also capable of requesting and receiving at least a portion of the medical-related information from the database. In addition, the system includes a controller capable of facilitating access to the database by the plurality of wireless nodes. The controller is also capable of detecting a new wireless node and communicating software to the new wireless node to allow the new wireless node to participate in the mesh network.

In another aspect, a controller includes a memory capable of storing a network protocol. The network protocol is to be used by nodes in a network to communicate. The controller also includes one or more processors collectively operable to identify a new node in the network, determine if the new node is capable of communicating using the network protocol, and provide the network protocol if the new node is not capable of communicating using the network protocol. The one or more processors are also collectively operable to facilitate access to a database by the new node using the network protocol to allow at least one of: storage of information from the new node in the database and delivery of information from the database to the new node.

In yet another aspect, a method includes identifying a new node in a network, determining if the new node is capable of communicating using a network protocol, and providing the network protocol if the new node is not capable of communicating using the network protocol. The method also includes facilitating access to a database by the new node using the network protocol to allow at least one of: storage of information from the new node in the database and delivery of information from the database to the new node.

Other technical features may be readily apparent to one skilled in the art from the following figures, descriptions, and claims.

### BRIEF DESCRIPTION OF THE DRAWINGS

For a more complete understanding of this disclosure, reference is now made to the following description, taken in conjunction with the accompanying drawings, in which:

FIGURE 1 illustrates an example system for wireless data communications according to one embodiment of this disclosure;

FIGURE 2 illustrates an example method for creating a wireless data communications network according to one embodiment of this disclosure;

FIGURE 3 illustrates an example method for wireless data storage according to one embodiment of this disclosure; and

FIGURE 4 illustrates an example method for wireless data retrieval according to one embodiment of this disclosure.

### DETAILED DESCRIPTION

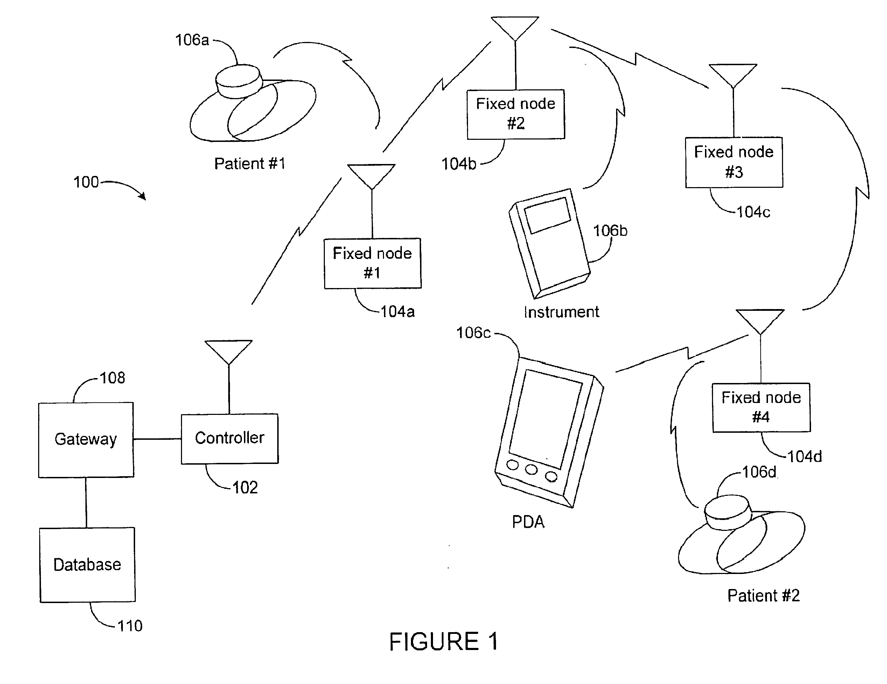

FIGURE 1 illustrates an example system 100 for wireless data communications according to one embodiment of this disclosure. In the illustrated embodiment, the system 100 includes a controller 102, fixed nodes 104a-104d (referred to as "fixed nodes 104"), portable nodes 106a-106d (referred to as "portable nodes 106"), a gateway 108, and a database 110. The system 100 shown in FIGURE 1 is for illustration only. Other embodiments of the system 100 may be used without departing from the scope of this disclosure.

The controller 102 is coupled to the gateway 108. In this document, the term "couple" and its derivatives refer to any direct or indirect communication between two or more elements, whether or not those elements are in physical contact with one another. The controller 102 supports the creation of a wireless network in the system 100. For example, the controller 102 may manage the addition and elimination of various nodes 104, 106 in the wireless network. Also, the controller 102 manages the flow of data to and from a gateway 108 that accesses a database 110. In some embodiments, a network protocol is implemented in software that is resident on the controller 102. As a particular example, the controller 102 may support the creation of a wireless mesh network.

The controller 102 includes any hardware, software, firmware, or combination thereof for communicating with one or more nodes 104, 106. For example, the controller 102 may represent a computer, such as a laptop or desktop computer, executing software that downloads instructions to the nodes 104, 106. In particular embodiments, the controller 102 includes one or more processors and one or more memories capable of storing instructions and data used by the one or more processors. The instructions downloaded to the nodes 104, 106 could include the entire network protocol, portions of the protocol such as updates and extensions, and parameters used by the protocol.

The fixed nodes 104 are capable of communicating with one another, portable nodes 106, and/or the controller 102. For example, a fixed node 104 may receive information from another fixed node 104, a portable node 106, or the controller 102. The fixed node 104 may then determine whether the information is meant for that node 104 or for another component in the system 100. As a particular example, the fixed node 104 may use a destination address associated with a message and determine if the node 104 has the same address. If meant for another component, the fixed node 104 forwards the information to another fixed node 104, a portable node 106, and/or the controller 102.

Each fixed node 104 includes any hardware, software, firmware, or combination thereof for receiving information from or communicating information to various components in the system 100. As an example, a fixed node 104 may include network hardware, such as radio frequency ("RF") hardware, and software downloaded from the controller 102 that provide a basic level of interconnectivity. In this document, the term "each" refers to every of at least a subset of the identified items.

The portable nodes 106 are capable of communicating with one another, fixed nodes 104, and/or the controller 102. For example, the portable nodes 106 may operate in a similar manner as the fixed nodes 104 described above, but the portable nodes 106 may move or roam within the system 100. The portable nodes 106 may also be customized for one or more specific applications, such as data collection and reporting functions, and act as network transfer agents. The specific application could represent patient or employee identification and monitoring. The specific application could also represent equipment identification, remote operation, and monitoring. In addition, the specific application could support access to medical-related data by a medical professional using a handheld appliance, such as a personal digital assistant ("PDA").

Each portable node 106 includes any hardware, software, firmware, or combination thereof for receiving information from or communicating information to various components in the system 100. As an example, a portable node 106 may include network hardware, such as RF hardware, and software downloaded from the controller 102 or a fixed node 104. In some embodiments, the portable nodes 106 are worn by patients or employees in a medical facility or are attached to medical instruments. A portable node 106 worn by a patient or employee could communicate information identifying the patient or employee and the location of the patient or employee. A patient's portable node 106 could also collect and communicate information about the medical condition of the patient. A portable node 106 attached to a medical instrument could include information identifying the instrument and the location of the instrument. In this example, the portable nodes 106 include portable patient monitors 106a, 106d (such as patient wristbands), a medical instrument 106b, and a personal digital assistant (PDA) 106c.

The gateway 108 facilitates access to the database 110. The gateway 108 includes any hardware, software, firmware, or combination thereof for supporting access to a database. For example, the gateway 108 could represent a primary computer system responsible for maintaining the database 110. The database 110 includes any hardware, software, firmware, or combination thereof for storing and facilitating retrieval of information. The database 110 may also use any of a variety of data structures, arrangements, and compilations to store and facilitate retrieval of information.

The controller 102, fixed nodes 104, and portable nodes 106 support the communication of medical-related data in the system 100. The medical-related data could, for example, represent information identifying a medical patient, the location of the patient, and the medical condition of the patient. The medical-related data could also represent information identifying an employee in a medical facility and the location of the employee. The medical-related data could further represent information identifying a medical instrument and the location of the medical instrument. In addition, the medical-related data could represent a patient's course of treatment, test results for a patient, current care for a patient, and a schedule for dispensing medication to a patient. Any other or additional medical data could be used in the system 100.

The medical-related data could be communicated to the controller 102 from the nodes 104, 106 for logging or storage in the database 110. The medical-related data could also be retrieved from the database 110 via the controller 102 and sent to the nodes 104, 106. In this way, various data can be stored and retrieved as needed by users in the system 100, such as by medical professionals using one or more of the nodes 104, 106. In particular embodiments, the data is accessible in real-time to health care professionals, such as doctors and nurses, in a hospital or other medical facility. The information provided in real-time could include a patient's treatment, test results, current care, or schedule for receiving medication.

In the system 100 illustrated in FIGURE 1, the nodes 104, 106 form a mesh network. A mesh network represents a random distribution of network nodes (nodes 104, 106), each of which may communicate with at least a neighboring node 104, 106 (such as its nearest neighbor). By means of a communication protocol stored locally at each node 104, 106, messages or other data can be communicated from any particular node 104, 106 to any other node 104, 106 in the network. Each node 104, 106 could also communicate with the controller 102, which may be viewed as a specialized node in the mesh network. The controller 102 provides long-term storage for the protocol software and manages network operation.

In some embodiments, the controller 102 and the nodes 104, 106 communicate wirelessly using RF signals. For example, a fixed node 104 or a portable node 106 may include an RF transceiver connected to a microcontroller that stores and executes mesh network software downloaded from the controller 102. The microcontroller also has access to data messages and addresses of destination nodes 104, 106.

The nodes 104, 106 may receive operating power in any suitable manner. For example, a fixed node 104 may acquire operating power from the utility services distributed through the facility in which the fixed node 104 is located, such as from the facility's electrical system. As another example, a portable node 106 may acquire operating power from a portable power source, such as a battery, fuel cell, or solar cell.

The fixed nodes 104 may be spaced apart in any suitable manner, such as by separating the nodes 104 so that one is within the operating range of its nearest neighboring node 104. This helps to provide basic operating coverage for the network. However, any other distribution of the fixed nodes 104 could be used.

In one aspect of operation, the controller 102 communicates with one or more neighboring fixed node(s) 104 to download software executed by the fixed node(s) 104. The software enables the operation of the mesh network. The software could include the network protocol, updates to the protocol, extensions to the protocol, and parameters used by the protocol. These fixed node(s) 104 then communicate the software to their neighboring fixed node(s) 104. This process may be repeated until all fixed nodes 104 are organized to provide a complete network. Also, as portable nodes 106 enter the network, the portable nodes 106 are cataloged, and software is downloaded (as required) to make the portable nodes 106 operational within the wireless network.

Data from one or more fixed nodes 104 and/or portable nodes 106 may be received by the controller 102 and stored in the database 110. The data may be received at the controller 102 directly from the nodes 104, 106 or indirectly (such as through one or more intermediate nodes 104, 106).

Stored data may be retrieved by an authorized user using any suitable device in the system 100. For example, a user using a portable node 106 could request data, and the request is sent directly or indirectly to the controller 102. The requested data is retrieved from the database 110 by the controller 102. The retrieved data is sent directly to the requesting user's device or indirectly to the user through one or more nodes 104, 106 of the network.

Data may be communicated between other nodes in the system 100 and need not only be transmitted between the nodes 104, 106 and the controller 102. For example, data originating at one portable node 106 may be communicated directly to another portable node 106 for use.

The network redundancy provided by overlapping ranges of the fixed nodes 104 and by the portable nodes 106 makes the network robust against the failure of any single node 104, 106. Moreover, the system 100 may be easily installed in one more facilities, and the system 100 may be easily integrated with existing wired communication systems.

Although FIGURE 1 illustrates one example of a system 100 for wireless data communications, various changes may be made to FIGURE 1. For example, the system 100 may include any number of fixed nodes 104 and portable nodes 106 in any suitable arrangement. Also, one or more of the fixed nodes 104 and/or the portable nodes 106 could represent devices that communicate with the controller 102 over a wireline connection. Further, the portable nodes 106 shown in FIGURE 1 are for illustration only. Other or additional types of portable nodes 106 could be used in the system 100. In addition, the database 110 could be coupled directly to the controller 102 without the use of a gateway 108.

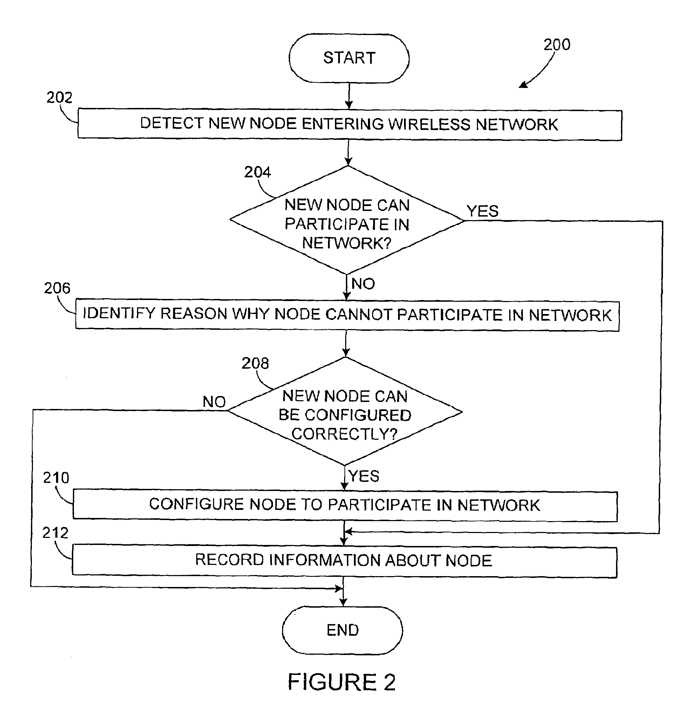

FIGURE 2 illustrates an example method 200 for creating a wireless data communications network according to one embodiment of this disclosure. For ease of explanation, the method 200 is described with respect to the controller 102 operating in the system 100 of FIGURE 1. The method 200 could be used by any other suitable device and in any other suitable system.

The controller 102 detects a new node at step 202. This may include, for example, the controller 102 detecting a new fixed node 104 or portable node 106 communicating directly with the controller 102. This may also include the controller 102 detecting a new fixed node 104 or portable 106 communicating indirectly with the controller 102 (such as through one or more nodes 104, 106 that have already been detected by the controller 102). The information received from the new node 104, 106 could represent any suitable information, such as a broadcast message identifying the new node 104, 106 that is periodically transmitted by the node 104, 106.

The controller 102 determines if the new node 104, 106 is capable of joining the wireless network managed by the controller 102 at step 204. This may include, for example, the controller 102 determining if the new node 104, 106 is using the appropriate software. The information identifying the software used by the new node 104, 106 may be retrieved in any suitable manner, such as by receiving a solicited or unsolicited message identifying the software.

If the new node 104, 106 can already participate in the wireless network, the controller 102 records information about the new node 104, 106 at step 212. This may include, for example, the controller 102 recording a network address associated with the new node 104, 106 so that the controller 102 may communicate with the new node 104, 106 as necessary.

If the new node 104, 106 cannot participate in the wireless network, the controller 102 determines if the new node 104, 106 can be configured to participate in the wireless network at step 608. This may include, for example, the controller 102 determining if the new node 104, 106 can be updated with software to participate in the wireless network. If the new node 104, 106 cannot be configured correctly, the method 300 ends. The new node cannot be used in or as part of the wireless network.

Otherwise, the controller 102 configures the new node 104, 106 at step 210. This may include, for example, the controller 102 communicating software for installation at the new node 104, 106. The software could include a network protocol, portions of the protocol such as updates and extensions, and parameters used by the protocol. The controller 102 may communicate directly with the new node 104, 106 or indirectly through one or more other nodes 104, 106. The controller 102 then records information about the new node 104, 106 at step 212.

Although FIGURE 2 illustrates one example of a method 200 for creating a wireless data communications network, various changes may be made to FIGURE 2. For example, other techniques could be used to allow new nodes 104, 106 to operate in a wireless network.

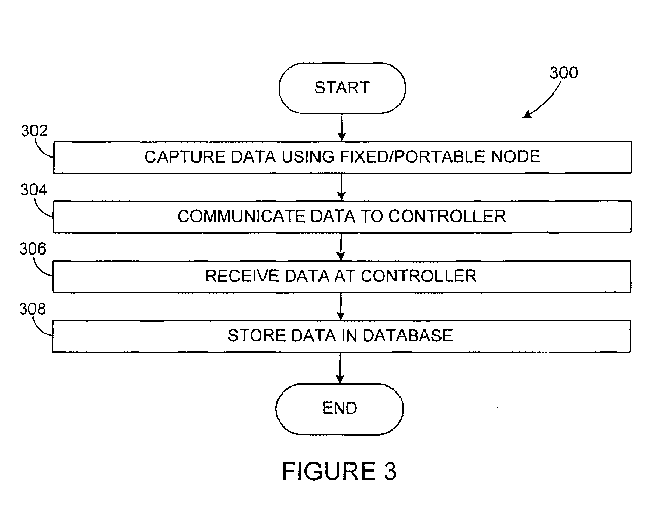

FIGURE 3 illustrates an example method 300 for wireless data storage according to one embodiment of this disclosure. For ease of explanation, the method 300 is described with respect to the system 100 of FIGURE 1. The method 300 could be used by any other suitable system.

A fixed node 104 or portable node 106 captures data at step 302. This may include, for example, a patient or employee monitor capturing information about the location and current condition of a patient or employee. This may also include an instrument monitor capturing information about the location of an instrument. This may further include a node receiving information from a user (such as typed information) identifying a diagnosis or course of treatment for a patient.

The information is communicated to the controller 102 at step 304. This may include, for example, the node 104, 106 communicating the information directly to the controller 102. This may also include the node 104, 106 communicating the information in directly to the controller 102 through one or more other nodes 104, 106.

The information is received at the controller 102 at step 306, and the controller 102 stores the information at step 308. This may include, for example, the controller 102 providing the information to a gateway 108 for storage in a database 110. This may also include the controller 102 storing the information directly in the database 110 without the use of a gateway 108.

Although FIGURE 3 illustrates one example of a method 300 for wireless data storage, various changes may be made to FIGURE 3. For example, some data may be stored locally by a node 104, 106 and not communicated to the database 110. Also, some data may be stored locally by a node 104, 106 and communicated to the database 110 at specified intervals or when polled.

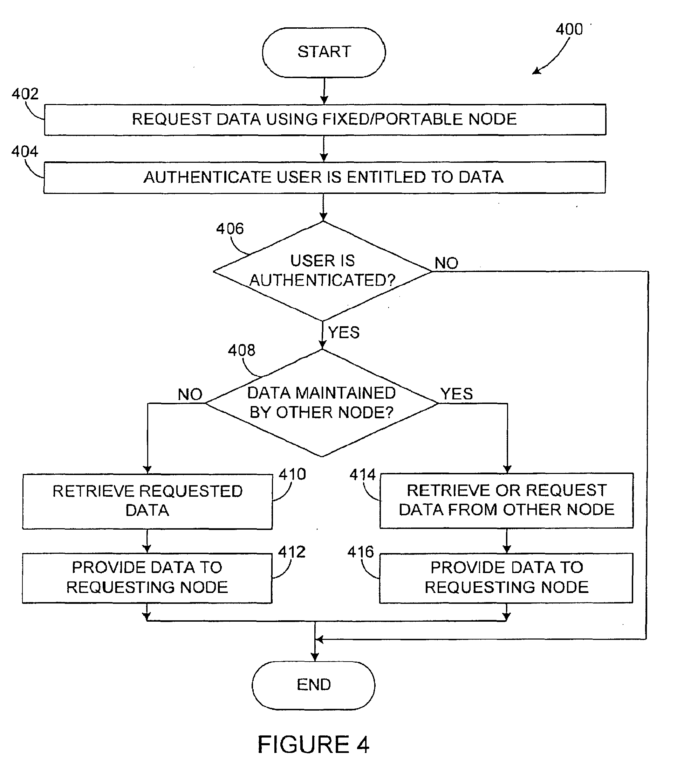

FIGURE 4 illustrates an example method 400 for wireless data retrieval according to one embodiment of this disclosure. For ease of explanation, the method 400 is described with respect to the system 100 of FIGURE 1. The method 400 could be used by any other suitable system.

Data is requested at a fixed node 104 or a portable node 106 at step 402. This may include, for example, a user using a fixed node 104, such as a computing device, to request particular data. This may also include the user using a portable node 106, such as a personal digital assistant, to request particular data.

An attempt to authenticate the user occurs at step 404. This may include, for example, the controller 102 determining if the user is authorized to retrieve the requested data. As particular examples, the user may have logged onto a node 104, 106 before requesting the data, and the controller 102 could use authentication information (such as a user name and password) provided when the user logged onto the node. As another particular example, the user could provide authentication information to the controller 102 when the user requests the data. Any other or additional technique could be used to authenticate the user.

If the user is not authorized to retrieve the requested data at step 406, the method 400 ends. At this point, the data is not provided to the user. Any suitable action could occur when the user fails to be authenticated, such as recording information about the user requesting the data, the node where the request for data originated, and any or additional information.

If the user is authorized to retrieve the requested data at step 406, a determination is made as to whether the requested data is stored in the database 110 or is being maintained by a node 104, 106 at step 408. This may include, for example, the controller 102 determining if the requested data is available in the database 110. This may also include the controller 102 polling other nodes 104, 106 if the requested data is not available.

If the requested data is available in the database 110, the data is retrieved at step 410. This may include, for example, the controller 102 retrieving the requested data from the database 110, either directly or indirectly through the gateway 108.

The retrieved data is provided to the node 104, 106 that requested the data at step 412. This may include, for example, the controller 102 providing the requested data directly to the node 104, 106 that requested the data. This may also include the controller 102 providing the requested data indirectly to the node 104, 106 that requested the data through one or more other nodes 104, 106.

If the requested data is being maintained by a node 104, 106 in the system 100, the requested data is either retrieved from that node or a request for the data is sent to the node at step 414. This may include, for example, the controller 102 retrieving the data from the node 104, 106 that is maintaining the data. This may also include the controller 102 sending a request to the node 104, 106 that is maintaining the data, where the request causes the node to provide the data to the requesting node.

The requested data is provided to the node 104, 106 that requested the data at step 416. This may include, for example, the controller 102 providing the data retrieved from the other node 104, 106 to the requesting node. This may also include the other node 104, 106 providing the data to the requesting node (either directly or indirectly).

Although FIGURE 4 illustrates one example of a method 400 for wireless data retrieval, various changes may be made to FIGURE 4. For example, the controller 102 could skip the authentication step if authentication is not needed for particular requested data or for all requested data. Also, the controller 102 may not be involved at all in data communications that occur between two nodes 104, 106.

It may be advantageous to set forth definitions of certain words and phrases used in this patent document. The terms "include" and "comprise," as well as derivatives thereof, mean inclusion without limitation. The term "or" is inclusive, meaning and/or. The phrases "associated with" and "associated therewith," as well as derivatives thereof, may mean to include, be included within, interconnect with, contain, be contained within, connect to or with, couple to or with, be communicable with, cooperate with, interleave, juxtapose, be proximate to, be bound to or with, have, have a property of, or the like. The term "controller" means any device, system, or part thereof that controls at least one operation. A controller may be implemented in hardware, firmware, or software, or a combination of at least two of the same. It should be noted that the functionality associated with any particular controller may be centralized or distributed, whether locally or remotely.

While this disclosure has described certain embodiments and generally associated methods, alterations and permutations of these embodiments and methods will be apparent to those skilled in the art. Accordingly, the above description of example embodiments does not define or constrain this disclosure. Other changes, substitutions, and alterations are also possible without departing from the spirit and scope of this disclosure, as defined by the following claims.

## Claims

1. A system, comprising:
a database capable of storing medical-related information;
a plurality of wireless nodes forming a mesh network, at least one of the plurality of wireless nodes capable of communicating at least a portion of the medical-related information for storage in the database, at least one of the plurality of wireless nodes also capable of requesting and receiving at least a portion of the medical-related information from the database; and
a controller capable of facilitating access to the database by the plurality of wireless nodes, the controller also capable of detecting a new wireless node and communicating software to the new wireless node to allow the new wireless node to participate in the mesh network.

2. The system of Claim 1, wherein the plurality of wireless nodes comprises at least one of: one or more fixed nodes and one or more portable nodes.

3. The system of Claim 2, wherein the one or more portable nodes comprise at least one of: a patient monitor, an employee monitor, a medical instrument, and a personal digital assistant.

4. The system of Claim 1, wherein the controller is capable of:
communicating directly with one or more first nodes of the plurality of wireless nodes; and
communicating indirectly with one or more second nodes of the plurality of wireless nodes through the one or more first nodes.

5. The system of Claim 1, further comprising a gateway coupled between the controller and the database, the gateway capable of maintaining the database.

6. The system of Claim 1, wherein the medical-related information comprises at least one of: an identity of a patient, a location of the patient, a medical condition of the patient, a course of treatment for the patient, a test result for the patient, a schedule for dispensing medication to the patient, an identity of an employee in a medical facility, a location of the employee, an identity of a medical instrument, and a location of the medical instrument.

7. The system of Claim 1, wherein the software communicated to the new wireless node comprises at least one of: a network protocol, an update to the network protocol, an extension to the network protocol, and a parameter for the network protocol.

8. A controller, comprising:
a memory capable of storing a network protocol, the network protocol to be used by nodes in a network to communicate; and
one or more processors collectively operable to:
identify a new node in the network;
determine if the new node is capable of communicating using the network protocol;
provide the network protocol if the new node is not capable of communicating using the network protocol; and
facilitate access to a database by the new node using the network protocol to allow at least one of: storage of information from the new node in the database and delivery of information from the database to the new node.

9. The controller of Claim 8, wherein:
the network protocol is implemented in software stored in the memory; and
the one or more processors are collectively operable to provide the network protocol by communicating the software.

10. The system of Claim 8, wherein the one or more processors are collectively operable to provide the network protocol by communicating software implementing at least one of: the network protocol, an update to the network protocol, an extension to the network protocol, and a parameter for the network protocol.

11. The controller of Claim 10, wherein:
the network comprises a mesh network; and
the one or more processors are collectively operable to provide the network protocol by at least one of:
communicating the software directly to the new node; and
communicating the software indirectly to the new node through one or more other nodes in the mesh network.

12. The controller of Claim 8, wherein the one or more processors are collectively operable to facilitate access to the database by communicating with a gateway, the gateway capable of maintaining the database.

13. The controller of Claim 8, wherein the nodes in the network comprise at least one of: one or more fixed nodes and one or more portable nodes.

14. The controller of Claim 13, wherein the one or more portable nodes comprise at least one of: a patient monitor, an employee monitor, a medical instrument, and a personal digital assistant.

15. The controller of Claim 8, wherein the information comprises at least one of: an identity of a patient, a location of the patient, a medical condition of the patient, a course of treatment for the patient, a test result for the patient, a schedule for dispensing medication to the patient, an identity of an employee in a medical facility, a location of the employee, an identity of a medical instrument, and a location of the medical instrument.

16. A method, comprising:
identifying a new node in a network;
determining if the new node is capable of communicating using a network protocol;
providing the network protocol if the new node is not capable of communicating using the network protocol; and
facilitating access to a database by the new node using the network protocol to allow at least one of: storage of information from the new node in the database and delivery of information from the database to the new node.

17. The method of Claim 16, wherein:
the network protocol is implemented in software stored in the memory; and
providing the network protocol comprises communicating the software.

18. The method of Claim 16, wherein providing the network protocol comprises communicating software implementing at least one of: the network protocol, an update to the network protocol, an extension to the network protocol, and a parameter for the network protocol.

19. The method of Claim 18, wherein:
the network comprises a mesh network; and
providing the network protocol comprises at least one of:
communicating the software directly to the new node; and
communicating the software indirectly to the new node through one or more other nodes in the mesh network.

20. The method of Claim 16, wherein facilitating access to the database comprises communicating with a gateway, the gateway capable of maintaining the database.

21. The method of Claim 16, wherein the nodes in the network comprise at least one of: one or more fixed nodes and one or more portable nodes.

22. The method of Claim 21, wherein the one or more portable nodes comprise at least one of: a patient monitor, an employee monitor, a medical instrument, and a personal digital assistant.

23. The method of Claim 16, wherein the information comprises at least one of: an identity of a patient, a location of the patient, a medical condition of the patient, a course of treatment for the patient, a test result for the patient, a schedule for dispensing medication to the patient, an identity of an employee in a medical facility, a location of the employee, an identity of a medical instrument, and a location of the medical instrument.

24. A computer program embodied on a computer readable medium and capable of being executed by a processor, the computer program comprising computer readable program code for:
identifying a new node in a network;
determining if the new node is capable of communicating using a network protocol;
providing the network protocol if the new node is not capable of communicating using the network protocol; and
facilitating access to a database by the new node using the network protocol to allow at least one of: storage of information from the new node in the database and delivery of information from the database to the new node.

25. The computer program of Claim 24, wherein:
the network protocol is implemented in software stored in the memory; and
the computer readable program code for providing the network protocol comprises computer readable program code for communicating the software.

26. The computer program of Claim 24, wherein the computer readable program code for comprises computer readable program code for communicating software implementing at least one of: the network protocol, an update to the network protocol, an extension to the network protocol, and a parameter for the network protocol.

27. The computer program of Claim 26, wherein:
the network comprises a mesh network; and
the computer readable program code for providing the network protocol comprises computer readable program code for at least one of:
communicating the software directly to the new node; and
communicating the software indirectly to the new node through one or more other nodes in the mesh network.

28. The computer program of Claim 24, wherein the computer readable program code for facilitating access to the database comprises computer readable program code for communicating with a gateway, the gateway capable of maintaining the database.

29. The computer program of Claim 24, wherein the nodes in the network comprise at least one of: one or more fixed nodes and one or more portable nodes.

30. The computer program of Claim 29, wherein the one or more portable nodes comprise at least one of: a patient monitor, an employee monitor, a medical instrument, and a personal digital assistant.

31. The computer program of Claim 24, wherein the information comprises at least one of: an identity of a patient, a location of the patient, a medical condition of the patient, a course of treatment for the patient, a test result for the patient, a schedule for dispensing medication to the patient, an identity of an employee in a medical facility, a location of the employee, an identity of a medical instrument, and a location of the medical instrument.
